(19) Europäisches Patentamt
European Patent Office
Office européen des brevets

(11) Publication number: **0 073 673**
**B1**

(12) **EUROPEAN PATENT SPECIFICATION**

(45) Date of publication of patent specification: **10.12.86**

(21) Application number: **82304558.8**

(22) Date of filing: **31.08.82**

(51) Int. Cl.[4]: **C 07 C 2/76,** C 07 C 2/84 // C07C19/02

(54) **Condensation of natural gas or methane into gasoline range hydrocarbons.**

(30) Priority: **01.09.81 US 298486**

(43) Date of publication of application:
**09.03.83 Bulletin 83/10**

(45) Publication of the grant of the patent:
**10.12.86 Bulletin 86/50**

(84) Designated Contracting States:
**BE DE FR GB IT NL**

(56) References cited:
**DE-C- 615 447**
**FR-A- 858 992**
**GB-A- 255 829**
**US-A-2 546 180**
**US-A-3 708 553**
**US-A-4 094 924**
**US-A-4 116 880**
**US-H- 944 010**

**JOURNAL OF THE AMERICAN CHEMICAL SOCIETY, vol. 90, no. 10, 8th May 1968, pages 2726-2727, Easton, USA G.A. OLAH et al.:**

(73) Proprietor: **Olah, George Andrew**
**2252 Gloaming Way**
**Beverly Hills California (US)**

(72) Inventor: **Olah, George Andrew**
**2252 Gloaming Way**
**Beverly Hills California (US)**

(74) Representative: **Szczuka, Jan Tymoteusz et al**
**Cruikshank & Fairweather 19 Royal Exchange Square**
**Glasgow G1 3AE Scotland (GB)**

(56) References cited:
**"Chemistry in super acids. I. Hydrogen exchange and polycondensation of methane and alkanes in GSO3H-SbF5 ("Magic Acid") solution. Protonation of alkanes and the intermediacy of CH5+ and related hydrocarbon ions. The high chemical reactivity of "Paraffins" in ionic solution reactions"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field

This invention relates to the direct conversion of natural gas or methane into gasoline-range hydrocarbons (i.e., synthetic transportation fuels or lower olefins) via catalytic condensation using superacid catalysts.

Background Art

The present state of art for production of synthetic fuels from either coal or natural gas (the two major possible raw materials) involves initial production of synthesis gas which is then either converted directly to hydrocarbons (Fischer-Tropsch) or converted first to methyl alcohol, which subsequently is converted into hydrocarbons (Mobil process):

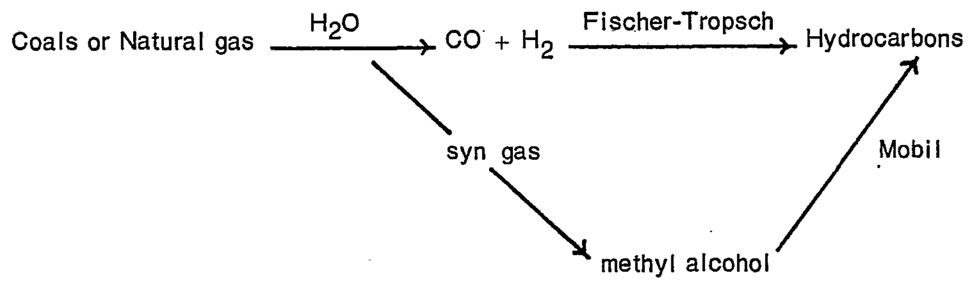


The Fischer-Tropsch process, although proven commercially, is not the most economically desirable process for the future due to its two very energetic steps and unsuitable product composition. The Mobil process is capable of producing gasoline-range hydrocarbons and aromatics relatively free of heavies, but suffers from the disadvantageous economics of first producing synthesis gas, which is then converted into methyl alcohol, which in turn is converted into hydrocarbons.

My discovery follows an independent and new route by utilizing methane (or natural gas) as the basic raw material. Methane as natural gas or even included as biological "deep methane" is expected to be available well into the 2000's, and, if not utilized exclusively as an energy source but rather for transportation fuels and as a chemical raw material source, could last much longer. Furthermore, coal can be readily converted into methane by methanation or by in-situ gasification, thus avoiding difficulties in mining and transporting coal. Further, alternate sources of methane, such as the biological conversion of biomass (sewage recycling, utilization of plant life on land and sea (algae or kelp farming of the sea) with subsequent off shore conversion allowing the piping of methane to land), are becoming available. If in the future as cheaper atomic or fusion energy becomes available, during off-peak periods, these plants could become producers of aluminium carbide which, upon hydrolysis, gives methane (with ethane and ethylene as by-products). The conversion of methane to higher hydrocarbons thus represents a viable new alternative to synthetic hydrocarbon processes.

The oligocondensation of methane was discovered by Olah et al., *Journal of the American Chemical Society, 90,* 2726 (1968), using exceedingly strong acid systems, the so-called "superacids", comprising a mixture of fluorosulfuric acid and antimony pentafluoride ("magic acid"), a mixture of hydrogen fluoride and antimony pentafluoride, or related superacids. Superacids have a Hammett acidity function $H_o$ less than — 11.9 $H_o$, the value for 100 percent sulfuric acid. However, yields were extremely low and the superacid was reductively depleted, rendering the process impractical on a commercial scale.

Alkane-alkene condensations (alkylations) such as that of isobutane with isobutylene to produce $C_8$ alkylate is well known in the petrochemical industry. Olah reported first the alkylation of alkanes with alkyl cations, generated in superacidic media (J.Am.Chem.Soc., *95*, 4939-4952 (1973)).

The condensation (polymerization) of methane and olefins represents a special problem and for a long time, methane was considered to be inert to usual acid catalyzed electrophilic reactions. The work of Olah on the superacid catalyzed reactivity of methanes opened up the possibility for such reactions. D. T. Roberts, Jr., and L. E. Calihan (J. Macronol. Sci-Chem., A7 (8) pp. 1641—1646 (1973)) reported that mixtures of methane and olefins (ethylene, propylene, etc.) polymerized in an autocalve at room temperature over a liquid magic acid ($HSO_3F/SbF_5$) catalyst to give oily oligomers with molecular weight of 100 to 700. Conversions were low (in the case of methane and ethylene, 5%) and under the used liquid phase conditions with long contact times (generally 24 hours) the olefin itself tended to polymerize on its own. Subsequently using another liquid superacid catalyst, hydrogen fluoride-tantalum pentafluoride, Siskin carried out the alkylation of methane with ethylene in a pressurized flow system (J.Am.Chem.Soc., *98*, 5413 (1976)). No yields or conversions were given and again the liquid phase reaction conditions were considered to substantially limit practical application of the reaction.

US—A—4 116 880 discloses a process for the heterogeneous gas-phase conversion of methane into gasoline-range hydrocarbons by condensing a gaseous feed containing methane and other lower alkanes in the presence of certain solid superacid catalysts. There is however no disclosure or suggestion of the use

of oxidative conditions and it is immediately apparent though that the rate of conversion achieved under the known (non-oxidative) conditions is very low, only 0.4% by weight of the methane being condensed to longer chain alkanes. Accordingly such a process would be very unattractive from the point of view of commercial production.

In fact the disclosure of US—A—4 116 880 is primarily concerned with providing new catalysts which had a *longer* effective life than previously known catalysts of the type. Thus the disclosure is concerned simply with providing an improved catalyst and not in any way concerned with improving the yield and conversion rate of any particular previously known process.

US—A—4 094 924 discloses the selective conversion of methane and other alkanes by condensation with $C_2$—$C_5$ olefins in the presence of a substantially liquid phase catalyst comprising a Lewis acid and a hydrogen halide to form $C_3$ and $C_4$—$C_8$ branched alkanes.

This publication does not disclose the use of oxidative conditions at all in the conversion of methane to gasoline range hydrocarbons. It also does *not* disclose the use of a solid superacid catalyst. On the contrary this publication specifically emphasizes the use of a substantially liquid phase catalyst. Such a catalyst would however give rise to considerable difficulties and expense in the fractionation of homogenous phase mixture in order to separate the catalyst from the desired products.

It is an object of the present invention to avoid or minimize one or more of the above disadvantages.

In sharp contrast to previous practice, I have now found a practical way to convert methane into gasoline-range hydrocarbons (synthetic transportation fuels) by condensing a gaseous feed comprising methane or natural gas in the presence of a solid or supported superacid.

The condensation of methane into higher hydrocarbons must overcome the unfavorable endothermic thermodynamics of such individual steps as noted below:

$$2CH_4 \longrightarrow C_2H_6 + H_2 + \text{kcal/mole}$$

In order to overcome this difficulty, it is necessary to oxidatively remove hydrogen. In accordance with the present invention higher hydrocarbons are produced when natural gas or methane is condensed in a single step in the presence of a suitable oxidant over the solid or supported superacid catalyst.

More particularly the present invention provides a process for the heterogeneous gas-phase conversion of methane into gasoline-range hydrocarbons (synthetic transportation fuels) by condensing a gaseous feed comprising methane or natural gas in the presence of a solid superacid catalyst characterized in that the gaseous feed is condensed in the presence of an oxidizing agent selected from air, oxygen, oxygen-ozone mixtures, sulfur, selenium, sulfur trioxide, nitrogen oxides and halogens.

The useful catalysts can be selected from higher valency Lewis Acid halides of metals of Groups IV, V and VI of the Periodic Table such as tantalum pentafluoride, niobium pentafluoride, antimony pentafluoride and the like, supported on a suitable chalconite carrier, preferentially in its fluorinated form such as fluorinated alumina or on a graphite, fluorinated graphite or aluminum trifluoride carrier.

As noted in Olah, G.A. "Friedel-Crafts Chemistry," N.Y., Wiley-Interscience, 1973. p. 343—344, the elements of Group VIA such as oxygen, sulfur, selenium or tellurium, have been called "chalcogens", and compounds containing these elements are called "chalconites", "chalcogenides" or "chalcides." A variety of solid oxides and sulfides, especially those comprising alumina, and mixtures of alumina, either natural or synthetic, in which other oxides such as chromia, magnesia, molybdena, thoria, tungstic oxide, zirconia, may also be present, as well as sulfides or molybdenum are useful chalcide carriers. Many naturally occurring compositions exist for use as the carrier including: bauxite, floridin, Georgia clay, and other natural aluminosilicates.

Synthetic chalcides, other than those of the silica-alumina type, representative of the chalcide carriers are: BeO, $Cr_2O_3$, $P_2O_5$, $ThO_2$, $TiO_2$, $Al_2(SO_4)_3$ (which may be regarded as $Al_2O_3$ $3SO_3$), $Al_2O_3$ $Cr_2O_3$, $Al_2O_3$, $Fe_2O_3$, $Al_2O_3$ CoO, $Al_2O_3$ MnO, $Al_2O_3$ $V_2O_3$, $Al_2O_3$ $Mo_2O_3$, $Cr_2O_3$ $Fe_2O_3$, $MoS_2$, and $MoS_3$.

The chalcide supports should be physically and chemically stable. Under the reaction conditions, they are generally catalytically active at only higher temperatures, as their acidity is not great enough to lead them to form stable complexes with unsaturated compounds, as do the aluminum halides, for example.

Useful catalysts can also be selected from superacidic conjugated acids composed of a strong Bronsted acid such as hydrogen fluoride, fluorosulfuric acid, perfluoroalkane-sulfonic acids of 1 to 18 carbon atom length, supported on a suitable solid carrier, or polymeric perfluorinated acids such as Nafion-H or copolymers of perfluorovinyl perfluorinated polymeric resinsulfonic acids and the like, in conjunction with a suitable Lewis Acid fluoride, such as those of the metals of Groups IV, V and VI of the Periodic Table.

The solid or supported solid catalysts are used at temperatures between about 15 and 250°C. and pressures of from 1 to 152 or more bar (1 to 150 or more atmospheres). The feed comprises methane alone, or mixtures which generally contain a substantial excess of methane, in mole ratios of 6 to 12:1 to olefins and/or acetylene, readily obtainable from pretreatment of natural gas by catalytic dehydrogenation or thermal reaction.

These condensations generally produce alkane mixtures, as well as cycloalkanes and aromatics of less than 12 carbons, but not olefins.

In accordance with the present invention an oxidizing agent selected from air, oxygen, oxyen-ozone mixture, sulfur, selenium sulfur trioxide, nitrogen oxides, halogens (fluorine, chlorine, bromine, iodine) is utilized to remove hydrogen as indicated below.

$$CH_4 \xrightarrow[-HX]{\text{oxidizing agent catalyst}} \text{hydrocarbons}$$

Oxidative condensation in the presence of air or oxygen gives water as a by-product. Recovery of hydrogen is thus not feasible.

The oxidative condensation of methane with sulfur gives $H_2S$ as a by-product. It can be readily oxidized to recover sulfur or, alternatively, it was discovered that by combining a CO shift reaction over a supported molybdenum sulfide catalyst with catalytic or thermal decarbonylation of COS, it is possible to obtain hydrogen and regenerate sulfur, thus allowing the following condensation:

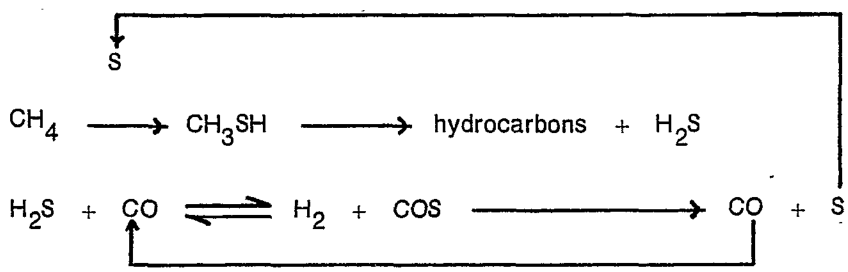


to proceed to hydrocarbons and hydrogen. This approach allows a methane condensation with the recovery of hydrogen, or is also applicable to hydrogen production from refinery gases, sour gas wells or coal desulfurization.

The oxidative condensation of methane can also be carried out with halogens as the oxidizing agents. Methane can be chlorinatively (brominatively, iodinatively) condensed under the reaction conditions. Initially, methyl chloride, bromide, or iodide are formed (or methyl fluoride, if the halogenation is carried out in the presence of HF) which are subsequently readily condensed into higher hydrocarbons. HCl, HBr or HI can be recycled via oxyhalogenation, whereas HF is reusable as such. The condensation reaction is preferably carried out at a temperature of about 50 to 250°C and at a pressure of from 1 to 51 bar (1 to 50 atmospheres).

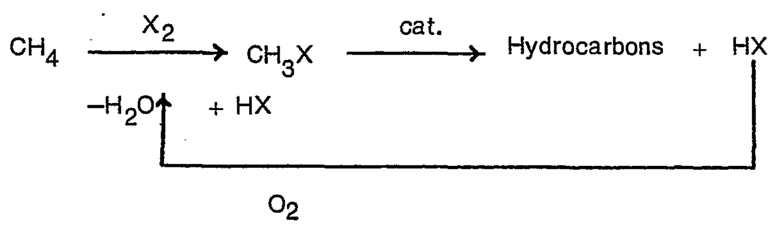


X = Cl, Br, I

Illustrative of the invention are the following examples. In the related Tables where product compositions are given, they have been normalized, even if not stated, to provide a total of 100% conversion, excluding unreacted methane which can be recycled.

Example 1

A 9:1 natural gas (consisting of about 90% methane and 10% ethane) — oxygen mixture was reacted at a pressure of 66 bar (65 atmospheres) at 120°C over a 20% antimony pentafluoride deposited on fluorinated graphite. A 13% conversion was obtained. The hydrocarbon product contained 21 to 27% ethylene, 6 to 8% propane, 31—40% butanes, 11—16% pentanes, with the balance being oxygenated products.

Example 2

Methane was reacted in the presence of sulfur in the ratio of 5:1 in the presence of the tantalum pentafluoride catalyst containing HF at 270°C in a stainless steel pressure reactor. A 2 to 10% conversion to condensed saturated hydrocarbons is achieved depending on reaction time (1 to 24 hrs) with the following product composition.

| Product composition | (%)[c] |
|---|---|
| ethane | 38—50 |
| propane | 5— 8 |
| butanes | 40—49 |
| pentanes | 5—15 |

Example 3

Methane was reacted in the presence of sulfur in a ratio of 5:1 in the presence of antimony pentafluoride catalyst at 200°C as in Example 2. A 3 to 15% conversion with similar produce composition was obtained.

Example 4

Methane was reacted in the presence of selenium in a ratio of 5:1 in the prsence of the tantalum pentafluoride catalysts at 200°C in a stainless steel pressure autoclave for six hours. A 3 to 16% conversion to condensed saturated hydrocarbons is achieved with the following typical product composition:

| Product composition | (%)[a] |
|---|---|
| ethane | 18—34 |
| propane | 2—5 |
| butanes | 34—48 |
| pentanes | 11—23 |
| hexanes | 6—19 |

Example 5

A 2:1 methane-chlorine mixture was reacted over a perfluorinated resinsulfonic acid (Nafion—H) catalyst complexed with 20% tantalum pentafluoride at 185°C in the previously utilized continuous flow reactor. 40% per pass conversion gave the following product composition:

| Product composition | (%)[a] |
|---|---|
| methyl chloride | 2 |
| methylene chloride | 7 |
| ethane | 3 |
| ethyl chloride | 40 |
| isobutane | 48 |

Example 6

A 2:1 methane-chlorine mixture was reacted over a catalyst prepared by intercalating (immobilizing) 30% antimony pentafluoride onto graphite, at 120°C. Typical per pass conversions were 32% with product composition of

| Product composition | (%)[c] |
|---|---|
| methyl chloride | 37—58 |
| ethyl chloride | 1—4 |
| methylene chloride | 9—14 |
| isobutane | 25—48 |

Example 7

In a pressure reactor, methane was reacted over a graphite catalyst intercalated with 30% antimony pentafluoride at 20°C and 9.66 bar (140 psi) pressure. The reaction was initiated with anhydrous HF and a halogen (fluorine, chlorine). Initial methane conversion was 15 to 29% with the following typical hydrocarbon product composition (excluding halomethanes):

| Product composition | (%)[a] |
|---|---|
| ethane | 8—23 |
| propane | 3—8 |
| butanes | 32—48 |
| pentanes | 11—28 |
| hexanes | 6—15 |

Conversion decreases with time as activity decreases during the reaction. It can, however, be restored by treating the system again with anhydrous hydrogen fluoride containing fluorine or chlorine as the oxidizing agent. The reaction is advantageously carried out using alternating parallel ractors, in one of which the reaction is carried out, while in the other system is regenerated.

Example 8

Methane was reacted over a catalyst, which was 20% tantalum pentafluoride supported on anhydrous aluminum fluoride, in a pressure reactor at 20°C and 9.66 bar (140 psi). The reaction is initiated with anhydrous hydrogen fluoride and a halogen (fluorine or chlorine). The reaction was carried out and the system reactivated as in example 7. Initial methane conversion was 12 to 19% with the following typical hydrocarbon product composition (excluding halomethanes):

| Product composition | (%)[a] |
|---|---|
| ethane | 8—19 |
| propane | 1—5 |
| butanes | 46—58 |
| pentanes | 12—27 |
| hexanes | 5—17 |

**Claims**

1. A process for the heterogeneous gas-phase conversion of methane into gasoline-range hydrocarbons (synthetic transportation fuels) by condensing a gaseous feed comprising methane or natural gas in the presence of a solid superacid catalyst characterized in that the gaseous feed is condensed in the prsence of an oxidizing agent selected from air, oxygen, oxygen-ozone mixtures, sulfur, selenium, sulfur trioxide, nitrogen oxides and halogens.

2. A process according to claim 1 wherein the solid superacid catalyst is selected from higher valency Lewis Acid halides of metals of groups IV, V and VI of the Periodic Table, supported on a carrier.

3. A process according to claim 2 wherein the higher valency Lewis Acid halide is selected from tantalum pentafluoride, niobium pentafluoride and antimony pentafluoride.

4. A process according to claim 2 or claim 3 wherein the carrier is a chalconite, graphite, fluorinated graphite or aluminum fluoride.

5. A process according to claim 4, wherein the fluorinated chalconite carrier is fluorinated.

6. A process according to claim 5, wherein the fluorinated chalconite carrier is fluorinated alumina.

7. A process according to claim 4 wherein the solid superacid catalyst is antimony pentafluoride deposited on graphite or fluorinated graphite.

8. A process according to claim 4 wherein the solid superacid catalyst is tantalum pentafluoride deposited on aluminum trifluoride, fluorinated alumina, graphite or fluorinated graphite.

9. A process according to claim 1 wherein the solid superacid catalyst is composed of a strong Bronsted acid component selected from fluorosulfuric acid, perfluoroalkanesulfonic acids of 1 to 18 carbon atoms deposited on a suitable carrier, perfluorinated polymeric resinsulfonic acids or copolymers of

perfluorovinylsulfonic acid and tetrafluoroethylene or trifluorochloroethylene complexed with a Lewis Acid fluoride of a metal selected from groups IV, V and VI of the Periodic Table.

10. A process according to any one of claims 1 to 9 wherein the gaseous feed is condensed at a temperature of from 15 to 250°C and a pressure of from 1 to 152 bar.

11. A process according to claim 10 wherein the gaseous feed is condensed at a temperature of from 50 to 250°C.

12. A process according to any one of claims 1 to 11 wherein the methane or natural gas is condensed in admixture with at least one lower olefin in the $C_2$ to $C_4$ range.

13. A process according to claim 12 which includes the preliminary step of treating natural gas by catalytic dehydrogenation so as to produce the mixture of methane or natural gas with at least one lower olefin in the $C_2$ to $C_4$ range.

14. A process according to any one of claims 1 to 11 wherein the methane or natural gas is condensed in admixture with acetylene.

15. A process according to claim 14 which includes the preliminary step of thermally treating methane or natural gas so as to produce a mixture comprising methane or natural gas and acetylene.

**Patentansprüche**

1. Verfahren für die heterogene Gasphasen-Umwandlung von Methan in Kohlenwasserstoffe im Benzinbereich (synthetische Transportbrennstoffe) durch Kondensieren einer gasförmigen Beschickung, die Methan oder Naturgas enthält, in Anwesenheit eines festen übersauren Katalysators, dadurch gekennzeichnet, daß die gasförmige Beschickung in Anwesenheit eines Oxidationsmittels aus der Gruppe Luft, Sauerstoff, Michungen von Sauerstoff und Ozon, schwefel, Selen, Schwefeltrioxid, Stickstoffoxiden und Halogenen kondensiert wird.

2. Verfahren nach Anspruch 1, wobei der feste übersaure Katalysator aus höherwertigen Lewissäure-Halogeniden von Metallen der Gruppen IV, V und VI des Periodensystems, auf einem Träger ausgewählt ist.

3. Verfahren nach Anspruch 2, worin das höherwertige Lewissäure-Halogenid Tantalpentafluorid, Niobpentafluorid und/oder Antimonpentafluorid ist.

4. Verfahren nach Anspruch 2 oder 3, worin der Träger ein Chalconit, Graphit, fluoriertes Graphit oder Aluminiumfluorid ist.

5. Verfahren nach Anspruch 4, worin der Chalconit-Träger fluoriert ist.

6. Verfahren nach Anspruch 5, wobei der fluorierte Chalconit-Träger fluoriertes Aluminiumdioxid ist.

7. Verfahren nach Anspruch 4, worin der feste übersaure Katalysator auf Graphite oder fluoriertem Graphit niedergeschlagenes Antimonpentafluorid ist.

8. Verfahren nach Anspruch 4, wobei der feste übersaure Katalysator auf Aluminiumtrifluorid, fluoriertem Aluminiumoxid, Graphit und fluoriertem Graphit niedergeschlagenes Tantalpentafluorid ist.

9. Verfahren nach Anspruch 1, wobei der feste übersaure Katalysator aus einer starken Bronstedsäure-Komponente, ausgewählt aus Fluoroschwefelsäure, Perfluoroalkansulfonsäuren mit 1 bis 18 Kohlenstoffatomen, niedergeschlagen auf einem geeigneten Träger, perfluorierten polymeren Harzsulfonsäuren oder Kopolymeren von Perfluorvinylsulfonsäure und Tetrafluoräthylen oder Trifluorchloräthylen, mit einem Lewissäure-Fluorid eines Metalls, das aus den Gruppen IV, V und VI des periodischen Systems ausgewählt ist, komplexartig gebunden ist.

10. Verfahren nach einem der Ansprüche 1 bis 9, wobei die gasförmige Beschickung bei einer Temperatur von 15 bis 250°C und einem Druck von 1 bis 152 bar kondensiert wird.

11. Verfahren nach Anspruch 10, wobei die gasförmige Beschickung bei einer Temperatur von 50 bis 250°C kondensiert wird.

12. Verfahren nach einem der Ansprüche 1 bis 11, wobei das Methan oder Naturgas in Mischung mit wenigstens einem niedrigeren Olefin im $C_2$—$C_4$-Bereich kondensiert wird.

13. Verfahren nach Anspruch 12, das die vorausgehende Stufe der Behandlung von Naturgas durch eine solche Dehydrogenierung umfaßt, daß das Gemisch von Methan oder Naturgas mit wenigstens einem niedrigen Olefin in dem $C_2$—$C_4$-Beriech erzeugt wird.

14. Verfahren nach einem der Ansprüche 1 bis 11, wobei das Methan oder Naturgas im Gemisch mit Acetylen kondensiert wird.

15. Verfahren nach Anspruch 14, das die Vorstufe einer solchen thermischen Behandlung von Methan oder Naturgas umfaßt, daß ein Gemisch, enthaltend Methan oder Naturgas und Acetylen erzeugt wird.

**Revendications**

1. Procédé pour la conversion hétérogène en phase gazeuse de méthane en hydrocarbures du type essence (carburant de transport synthétique) par condensation d'une charge gazeuse comprenant du méthane ou un gaz naturel en présence d'un catalyseur solide superacide, caractérisé en ce que la charge gazeuse est condensée en présence d'un agent oxydant choisi parmi l'air, l'oxygène, les mélanges oxygène-ozone, le soufre, le sélénium, l'anhydride sulfurique, les oxydes d'azote et les halogènes.

2. Procédé selon la revendication 1, dans lequel le catalyseur solide superacide est sélectionné parmi

les halogénures d'acides de Lewis à valence supérieure des métaux des groupes IV, V et VI de la Classification Périodique, supportés sur un support.

3. Procédé selon la revendication 2, dans lequel l'halogénure d'acides de Lewis à valence supérieure est sélectionné parmi le pentafluorure de tantale, le pentafluorure de niobium et le pentafluorure d'antimoine.

4. Procédé selon la revendication 2 ou la revendication 3, dans lequel le support est une chalconite, du graphite, du graphite fluoré ou du fluorure d'aluminium.

5. Procédé selon la revendication 4, dans lequel le support de chalconite est fluoré.

6. Procédé selon la revendication 5, dans lequel le support en chalconite fluoré est de l'alumine fluorée.

7. Procédé selon la revendication 4, dans lequel le catalyseur solide superacide est du pentafluorure d'antimoine déposé sur du graphite ou du graphite fluoré.

8. Procédé selon la revendication 4, dans lequel le catalyseur solide superacide est le pentafluorure de tantale déposé sur du trifluorure d'aluminium, de l'alumine fluorée, du graphite ou du graphite fluoré.

9. Procédé selon la revendication 1, dans lequel le catalyseur solide superacide est constitué par un composant d'acides forts de Brönsted, sélectionné parmi l'acide fluorosulfurique, les acides perfluoroalcanesulfoniques ayant 1 à 18 atomes de carbone déposés sur un support approprié, les acides polymères de résines sulfoniques perfluorés ou les copolymères de l'acide perfluorovinylsulfonique et de tétrafluoréthylène ou de trifluorochloréthylène complexés avec un fluorure d'acide de Lewis d'un métal sélectionné parmi les groupes IV, V et VI de la Classification Périodique.

10. Procédé selon l'une quelconque des revendications 1 à 9, dans lequel le charge gazeuse est condensée à une température allant de 15 à 250°C et à une pression allant de 1 à 152 bars.

11. Procédé selon la revendication 10, dans lequel la charge gazeuse est condensée à une température allant de 50 à 250°C.

12. Procédé selon l'une quelconque des revendications 1 à 11, dans lequel le méthane ou le gaz naturel sont condensés en mélange avec au moins une oléfine inférieure du domaine de $C_2$ à $C_4$.

13. Procédé selon la revendication 12 qui comprend l'étape préliminaire de traitement du gaz naturel par déshydrogénation catalytique afin de produire le mélange de méthane ou de gaz natural avec au moins une oléfine inférieure du domaine de $C_2$ à $C_4$.

14. Procédé selon l'une quelconque des revendications 1 à 11, dans lequel le méthane ou le gaz naturel sont condensés en mélange avec de l'acétylène.

15. Procédé selon la revendication 14 qui comprend l'étape préliminaire de traitement thermique du méthane ou du gaz naturel afin de produire un mélange comprenant du méthane ou du gaz naturel et de l'acétylène.